# EUROPÄISCHE PATENTSCHRIFT

(11) **EP 1 824 586 B1**
(45) Veröffentlichungstag und Bekanntmachung des Hinweises auf die Patenterteilung: **16.11.2011**
(21) Anmeldenummer: 05817596.9
(22) Anmeldetag: 03.12.2005
(51) Int. Cl.: B01D 67/00, B01D 69/00, G01N 33/52

(54) **VERFAHREN ZUR BESCHICHTUNG VON MEMBRANEN**
METHOD FOR COATING MEMBRANES
PROCEDE POUR ENDUIRE DES MEMBRANES

(30) Priorität: 07.12.2004 DE 102004058794
(43) Veröffentlichungstag der Anmeldung: 29.08.2007
(73) Patentinhaber: Roche Diagnostics GmbH, 68305 Mannheim (DE); F. Hoffmann-La Roche AG, 4070 Basel (CH)
(72) Erfinder: KLEPP, Juergen, 76676 Graben-Neudorf (DE); MANGOLD, Dieter, 67133 Maxdorf (DE); LERCH, Rolf, 68549 Ilvesheim (DE); FISCHER, Thomas, 69231 Rauenberg (DE); SCHAEFFLER, Juergen, 69469 Weinheim (DE)
(74) Vertreter: Rauscher, Andreas
(86) Internationale Anmeldenummer: PCT/EP2005/012959
(87) Internationale Veröffentlichungsnummer: WO 2006/061152

(56) Entgegenhaltungen:
- EP-A- 0 016 387
- EP-A- 0 654 659
- DE-A1- 4 025 768
- US-A- 4 824 639
- US-A- 5 066 398
- US-A- 5 169 787
- US-A- 5 213 689
- US-A- 5 536 470

## Beschreibung

Die Erfindung betrifft ein Verfahren zum Aufbringen feststofflialtiger Reaktivfilme auf mikroporöse Membranen, entsprechend hergestellte Membranen und sie enthaltende diagnostische Mittel.

Zur qualitativen oder quantitativen analytischen Bestimmung von Bestandteilen von Flüssigkeiten, insbesondere von Körperflüssigkeiten wie Blut, werden oft so genannte trägergebundene Tests verwendet. Bei diesen sind Reagenzien, insbesondere spezifische Nachweisreagenzien und Hilfsreagenzien, in entsprechenden Schichten eines festen Trägers eingebettet oder fixiert. Diese Schichten werden als Nachweiselemente bezeichnet. Zur Bestimmung des entsprechenden Analyten wird die flüssige Probe mit diesen Nachweiselementen in Kontakt gebracht. Die Reaktion von flüssiger Probe und den zunächst in trockener Form vorliegenden, durch die Probe wieder aufgelösten Reagenzien führt bei Anwesenheit eines Zielanalyten in der Regel zu einem optisch oder elektrochemisch nachweisbaren Signal, insbesondere einem Farbumschlag, welcher visuell oder mit Hilfe eines Geräts, meist reflexionsphotometrisch, ausgewertet werden kann. Andere Nachweismethoden beruhen beispielsweise auf elektrochemischen Methoden und erfassen Ladungs-, Potential- oder Stromänderungen.

Da die Nachweisreagenzien - anders als bei herkömmlichen Labortests - anfangs in trockener Form vorliegen, spricht man bei trägergebundenen Tests oft auch von "trockenchemischen Tests".

Testelemente oder Testträger für trockenchemische Tests sind häufig als Teststreifen ausgebildet, die im Wesentlichen aus einer länglichen Tragschicht aus Kunststoffmaterial und darauf angebrachten Nachweiselementen als Testfeldern bestehen. Es sind jedoch auch Testträger bekannt, die als quadratische oder rechteckige Plättchen gestaltet sind.

Der photometrische Nachweis niedermolekularer Analyte aus Blut mittels trockenchemischer Teststreifen beinhaltet üblicherweise die Abtrennung der die photometrische Messung störenden Erythrozyten.

Die zum Analytnachweis benötigten Enzyme befinden sich üblicherweise in einem wasserfesten, unlöslichen Film, bei dem eine hydrophobe Matrix bestehend aus Filmbildnern alle oder zumindest einen Teil der Nachweisreagenzien (d. h. im Wesentlichen Enzyme und Indikatorsystem) enthält, in die die Probe eindringt und in dem die farbbildende Reaktion stattfindet. Diese Filme werden mittels verschiedener etablierter Beschichtungsverfahren (z. B. Rakeln) auf nicht-saugende, mechanisch stabile Trägermaterialien (wie z. B. Pokalon^{®}-Folie aus Bisphenol-A-Polycarbonat) aufgebracht.

Unter dem Begriff Filmbildner werden Polymere verstanden, die die Beschichtung mechanisch stabiler, wasserfester Reagenzschichten erlauben (z. B. Propiofan^{®}, eine Vinylpropionat-Kunststoff-Dispersion).

Des Weiteren enthalten diese Reaktivfilme üblicherweise Quellstoffe. Quellstoffe sind wasserlösliche Polymere, die die Viskosität der Beschichtungsmasse maßgeblich beeinflussen, die die feine Verteilung der Reagenzien in den hydrophoben Teilbezirken der wasserfesten Schicht bewirken und die das Eindringen der Probe in die Schicht erleichtern (als Beispiel seien genannt Alginat, Keltrol®, Gantrez®, Eudragit, etc.).

Die "Offenporigkeit" und damit das Eindringverhalten von Analyt in den Reaktivfilm kann durch den Zusatz von Füllstoffen (auch "Filmöffner" genannt) positiv beeinflusst werden (vgl. hierzu z. B. US 4,312,834). Füllstoffe sind optisch nicht oder wenig streuende, wasserunlösliche, nicht quellende, gut benetzbare, feine, anorganische oder organische Partikel, die das schnelle Eindringen auch größerer Moleküle (beispielsweise Lipide in Form von Lipoproteinen) und sogar von Zellen (z. B. Erythrozyten) in wasserfeste Filme ermöglichen. Als Beispiel für Füllstoffe seien genannt Kreide, Cellulose, Diatomeenerde, Celatom, Kieselgur, Kieselsäure, etc.

Bei der ersten Generation von Blutglucose-Teststreifen (z. B. Hämoglukotest 20-800 von Boehringer Mannheim, vgl. auch US 3,630,957) enthielt der Reaktivfilm neben der Nachweischemie lediglich einen Filmbildner (Propiofan^{®}) und einen Quellstoff (Alginat). Bei diesen sehr dichten, d. h. wenig offenporigen, abwischfesten Filmen können Erythrozyten nicht in den Reaktivfilm eindringen, wohl aber niedermolekulare Blutinhaltsstoffe wie insbesondere Glucose. Eine separate Blutabtrennung war daher nicht notwendig. Der Blutstropfen, aus dem die Blutglukose bestimmt werden sollte, wurde einfach direkt auf den Reaktivfilm des Teststreifens aufgegeben. Nach einer Minute Inkubation des Blutstropfens auf dem Reaktivfilm wurde das Blut abgewischt, nach einer weiteren Minute Reaktionszeit konnte die Farbentwicklung als Maß der Analytkonzentration von der selben Seite des Steifens abgelesen werden, auf der zuvor das Blut aufgebracht worden war.

Damit war es erstmals möglich, Glucose direkt aus Vollblut nachzuweisen. Da diese Reaktivfilme keine Füllstoffe enthielten, gestatteten sie lediglich das langsame Eindringen niedermolekularer gut wasserlöslicher Analyte wie Glucose, nicht jedoch den Nachweis großer und hydrophober Moleküle (wie z. B. Cholesterin (CHOL), HDL (High density lipoprotein, d. h. Lipoproteine hoher Dichte), Triglyceride (TG), Creatinkinase (CK), etc.).

Ein Meilenstein in der Entwicklung trockenchemischer Teste zum Analytnachweis aus Vollblut erbrachte die Verwendung von Glasfaservliesen zur Erythrozytenabtrennung (siehe hierzu u. a. US 4,816,224), speziell in Kombination mit offenporigen, Füllstoffe enthaltenden Reaktivfilmen (z. B. die Teststreifen der Reflotron-Produktlinie von Roche Diagnostics und später die so genannten "Non-Wipe-Teste" der Accutrend-Linie von Roche Diagnostics). Neben deutlich schnelleren Kinetiken - insbesondere bezüglich des Eindringens des Analyten in den Nachweisfilm, enzymatischer Umsetzung und Farbreaktion - ermöglichten diese Testaufbauten auch den Nachweis relativ großer, hydrophober Moleküle (z. B. CHOL, HDL, TG, etc.).

Ein Nachteil der Glasfaservliestechnologie liegt jedoch im relativ ungünstigen Verhältnis des Volumens des nutzbaren Plasmas zum eingesetzten Blutvolumen (nachfolgend auch als Blut/Plasma-Ausbeute bezeichnet). Des Weiteren erwies sich beim oxidativen Analytnachweis (d. h. beim Analytnachweis mittels Analytoxidase und Umsetzung des dabei gebildeten Wasserstoffperoxids mit Peroxidase in Gegenwart eines Indikators, der dabei von einer (meist farblosen) reduzierten in eine (meist farbige) oxidierte Form übergeht) insbesondere in so genannten Stapelaufbauten (Glasfaservlies zum Abtrennen der Erythrozyten und Reaktivfilm bilden einen Stapelverbund; auf das Glasfaservlies wird die Blutprobe aufgegeben, diese durchdringt unter Abtrennung der roten Blutzellen das Glasfaservlies und das so erzeugte Serum bzw. Plasma dringt in die darunter liegende Reaktivfilmschicht ein, wo die eigentliche Nachweis- und Indikatorreaktion stattfindet, die dann von der der Blutauftragsseite gegenüberliegenden Seite des Stapelverbunds beobachtet werden kann) die Versorgung des Reaktivfilms mit Sauerstoff als Limitation, so dass nur ein nach oben hin eingeschränkter Messbereich erzielbar war.

Zur Minimierung des Blutvolumens verwendet daher die jüngste Generation an Teststreifen blutabtrennende Membranen (vgl. bspw. EP-A 0 654 659) bzw. sehr dünne Ein- oder Zwei-Schicht-Filme (vgl. hierzu US 5,536,470 und US 6,036,919). Die Blut/Plasma-Ausbeute ist für solche membranbasierenden Systeme in der Regel deutlich vorteilhafter als dies mit der Glasfasertechnologie der Fall ist. Beide membranbasierenden Systeme sollen nachfolgend erläutert werden.

US 5,536,470 offenbart Testfelder, die aus einer dünnen Filmschicht bestehen. Bei ihnen wird eine Vollblutprobe von einer Seite der Filmschicht aufgegeben. Eine Farbreaktion kann von der gegenüberliegenden Seite detektiert werden, ohne dass Erythrozyten von der Probenaufgabenseite zur Nachweisseite durchdringen können. Die Filmschicht kann auf einem transparenten Träger (z. B. Folie) oder einer Membran beschichtet sein. Die in US 5,536,470 offenbarte Filmschicht fungiert daher als kombinierte Blut(farbstoff)abtrenn- und Nachweisschicht. Zur Erfüllung der erstgenannten Funktion (Blut(farbstoff)abtrennung) ist ein hoher Pigmentanteil notwendig, d. h. der Pigmentgehalt beträgt hier mindestens 30 Gewichtsprozent bezogen auf den Feststoffgehalt der filmbildenden Masse. Zur Gewährleistung der mechanischen Stabilität solcher Filmschichten mit hohem Pigmentanteil ist auch ein hoher Gehalt an Filmbildner erforderlich. Pigment und Filmbildner sollten in annähernd gleichem Gewichtsverhältnis vorliegen. Inerte Füllstoffe (d. h. sogenannte Filmöffner) sollten in diesen Filmschichten möglichst gar nicht oder wenn, dann nur in sehr geringen Mengen (weniger als 10 % des gesamten Feststoffgehalts) in der filmbildenden Masse enthalten sein, da andernfalls die blutabtrennende Eigenschaft der Filmschicht nicht mehr gewährleistet ist. Aufgrund des geringen Füllstoffgehalts in der filmbildenden Masse von maximal 10 % sind die in US 5,536,470 offenbarten Filme allerdings nicht offenporig genug, um für große, hydrophobe Analyte (z. B. Lipide) durchlässig zu sein.

Beim Glucose-Nachweis mittels dünner Zwei-Schicht-Filme auf transparenter Folie enthält die Erstschicht (d. h. die Schicht, die direkt auf der Folie aufliegt) als Reaktivfilm neben dem Enzym-Indikator-System Filmbildner, Quellstoffe und einen optisch transparenten Füllstoff (z. B. Transpafill®, ein Natrium-Aluminium-Silikat von Degussa). In Analogie zu einem nasschemischen Photometernachweis bildet die transparente Erstschicht quasi die Küvette, in der der photometrische Analytnachweis abläuft. Die auf die Erstschicht aufgebrachte Zweitschicht enthält einen hohen Anteil eines hoch brechenden Pigments (z. B. Titandioxid) unter Verzicht auf Filmöffner oder Füllstoffe. Der Blutauftrag erfolgt direkt auf die Zweitschicht, die photometrische Detektion erfolgt von der gegenüberliegenden Teststreifenseite durch die transparente Trägerfolie in der Erstschicht.

Die optisch deckende, wenig offenporige Zweitschicht erfüllt dabei eine Doppelfunktion. Einerseits verhindert sie als blutabtrennender Film das Eindringen von Erythrozyten in die reaktive Erstschicht, andererseits reflektiert sie das durch die Erstschicht fallende Licht und verhindert ein Durchscheinen der roten Erythrozytenfarbe zur Detektionsseite.

Der Vorteil eines solchen Systems, verglichen zur Erythrozytenabtrennung mittels Glasfaservlies, liegt im geringen benötigten Probevolumen und in einer schnellen Kinetik beim Nachweis niedermolekularer Analyte.

Der Nachteil dieses Zwei-Schicht-Aufbaus liegt darin, dass große hydrophobe Moleküle (z. B. Lipoproteine, Cholesterin, Trigylceride, HDL, etc.) nicht durch die blutabtrennende Zweitschicht diffundieren und damit nicht in der Erstschicht nachgewiesen werden können.

Eine Alternative stellt daher die Verwendung blutabtrennender Membranen dar. Blutabtrennende (d. h. Plasma bzw. Serum aus Vollblut generierende) Membranen sind stark asymmetrische Membranen (üblicherweise Polyether oder Polyethersulfon, z. B. BTS-SP-300 der Fa. Pall, PrimeCare X oder SG der Fa. Spectral Diagnostics), d. h. Membranen, deren Porendurchmesser nicht gleichmäßig ist, sondern die eine offenporige und eine engporige Seite aufweisen. Der Blutauftrag erfolgt in der Regel auf die offenporigere Membranseite. Die Erythrozyten werden in den sich verjüngenden Poren beim Durchtritt des Probenmaterials durch die Membran zurückgehalten (vgl. hierzu EP 0 654 659).

Prinzipiell werden blutabtrennende Membranen in zwei Formen in trockenchemischen Teststreifen verwendet. Im so genannten Ein-Schicht-Aufbau erfüllt die blutabtrennende Membran neben der Blutabtrennung auch die Funktion des Trägers für die Nachweischemie. Zu diesem Zweck wird die Membran aus einem wässrigen, Indikator und Nachweissystem enthaltenden System getränkt (z. B. mittels Wannentränkung oder Schlitzdüsendosierung).

Um ein schnelles Lösen der eingetränkten und getrockneten Enzyme und ein schnelles Benetzen der Membran durch das Probenmaterial zu gewährleisten, werden der Tränklösung üblicherweise Netzmittel zugesetzt.

Ein Nachteil des Ein-Schicht-Membranaufbaus besteht darin, dass die Membran im trockenen Zustand zwar optisch intransparent ist (der Brechungsindex von Luft ist etwa 1,00; der Brechungsindex der Membran liegt etwa bei 1,35 -1,38, d. h. der Unterschied der Brechungsindices beträgt etwa 0,35 - 0,38, so dass die Membran intransparent erscheint), im feuchten Zustand jedoch optisch deutlich transparenter wird (der Brechungsindex von Wasser ist etwa 1,33, so dass der Unterschied der Brechungsindices nur noch etwa 0,02 - 0,05 beträgt) und damit die Bluteigenfarbe der in den unteren Membranbezirken separierten Erythrozyten durchscheint und die photometrische Messung beeinflusst.

Durch Zusatz von Weißpigmenten (z. B. Titandioxid, Brechungsindex etwa 2,55) in die Imprägnierlösung kann dies minimiert bis verhindert werden. Da optisch deckende Weißpigmente Partikelgrößen im Bereich der halben Wellenlänge des zu reflektierenden Lichts (0,2 bis 0,4 µm) besitzen, können sie beim Imprägnieren der Membran in die Poren (die typischerweise Durchmesser von 0,2 bis 10 µm haben) eindringen, diese dabei verengen bzw. blockieren und so den Eintritt und Durchtritt großer hydrophober Moleküle durch die Membran erschweren bzw. unmöglich machen.

Ein-Schicht-Aufbauten mit blutabtrennenden Membranen werden daher ausschließlich zum Nachweis kleiner, gut wasserlöslicher Analyte (z. B. Glucose) verwendet.

Ein Zwei-Schichten-Membranaufbau eröffnet die Möglichkeit, viele Probleme des Ein-Schichten-Aufbaus zu umgehen. In diesem Fall liegt benachbart zur blutabtrennenden Membran eine weitere, engporigere, das Plasma der blutabtrennenden Membran aufnehmende Nachweismembran (z. B. Biodyne A oder Loprodyne = 0,2 / 0,45 µm Nylonmembran der Fa. Pall). In diesem Fall werden keine optisch deckenden Weißpigmente benötigt. Des Weiteren tritt das in der zweiten Membran enthaltene Nachweissystem nicht in direkten Kontakt mit dem blutabtrennenden System, was insbesondere im Umfeld Lipidnachweis die Verwendung von Lipide gut anlösenden aber auch hämolytisch wirkenden Netzmitteln ermöglicht.

Der Zwei-Schichten-Aufbau birgt allerdings auch die Nachteile eines aufwändigen, teuren Testaufbaus. Prozesstechnisch werden hohe Anforderungen an die maschinelle Teststreifenmontage gestellt, da zum Serum- bzw. Plasmaübertritt aus der blutabtrennenden Membran in die Nachweismembran ein möglichst lückenloser, enger Kontakt gewährleistet sein muss. Systemimmanente Nachteile liegen in einer langsameren Benetzung des Testaufbaus, einer im Vergleich zum Ein-Schicht-Aufbau ungünstigeren Blut/PlasmaAusbeute und bedingt durch die engeren Poren der Nachweismembran einer langsameren Kinetik.

Nachteilig an den Verfahren des Standes der Technik ist also zusammenfassend, dass insbesondere für den Nachweis großer, hydrophober Moleküle offene Nachweisfilme mit hohem Füllstoffanteil erforderlich sind, solche offenen Nachweisfilme für sich genommen allerdings keine Abtrennung störender Blutbestandteile (v. a. Erythrozyten, Hämoglobin) für optisch auszuwertende Teststreifen gewährleisten. Geeignete blutabtrennende Systeme (Filme, Membranen) erlauben hingegen das Eindringen von großen hydrophoben Molekülen wenn überhaupt dann nur in ungenügendem Maße. Prinzipiell zum Nachweis großer, hydrophober Moleküle geeignete Systeme (wie z. B. die Kombination von Glasfaservlies und offenem Nachweisfilm oder von Zwei-Schicht-Membranaufbauten) lösen das Problem nur unbefriedigend, da sie von der Herstellung her kompliziert und störanfällig sind und in der Testperformance suboptimal sind (große benötigte Blutvolumina, nach oben hin limitierter Messbereich bzw. langsame Reaktionskinetik).

Aufgabe der vorliegenden Erfindung ist es, diese Nachteile zu beseitigen. Insbesondere ist es die Aufgabe der vorliegenden Erfindung, ein trockenchemisches Testdevice (sowie ein entsprechendes Herstellverfahren hierfür) bereitzustellen, mit dem es möglich ist, große, hydrophobe Moleküle, insbesondere Lipide, die in biologischen Proben als Lipoproteinkomplexe vorliegen, aus möglichst kleinen Mengen Vollblut nachzuweisen, wobei im Device eine Abtrennung von roten Blutzellen integriert ist und wobei eine schnelle Kinetik der Nachweisreaktion erreicht wird.

Diese Aufgabe wird durch den Gegenstand der Erfindung gelöst.

Gegenstand der Erfindung ist ein Verfahren gemäß Anspruch 1, mit Reaktivfilmen beschichtete mikroporöse Membranen mit hohem relativem Füllstoffanteil im film (bezogen auf den Filmbildner) gemäß Anspruch 7, sowie ein diagnostisches Mittel enthaltend eine erfindungsgemäße Membran gemäß Anspruch 9. Bevorzugte Ausführungsformen der Erfindung sind Gegenstand der abhängigen Ansprüche.

Das erfindungsgemäße Verfahren zum Aufbringen feststoffhaltiger Reaktivfilme auf mikroporöse Membranen zeichnet sich dadurch aus, dass die Membran zunächst befeuchtet und der feststoffhaltige Reaktivfilm auf die noch feuchte Membran aufgebracht wird.

Feststoffhaltige Reaktivfilme sind wasserfeste, wasserunlösliche Filme, die in einer hydrophoben Matrix aus Filmbildnern alle oder zumindest Teile der Nachweisreagenzien enthalten. Neben der eigentlichen Nachweischemie, die typischerweise Enzyme, Co-Enzyme, Mediatoren, Indikatoren bzw. Indikatorsysteme etc. umfasst, können Reaktivfilme wasserfeste Filmbildner, Filmöffner und gegebenenfalls optisch sperrende Pigmente (letztere zum Herabsetzen der optischen Transparenz) sowie weitere, dem Fachmann bekannte Bestandteile (Netzmittel, Quellstoffe, etc.) enthalten.

Als Filmöffner (auch als "Füllstoffe" bezeichnet) kommen erfindungsgemäß anorganische oder organische, insbesondere partikelförmige Materialien in Frage. Solche Filmöffner sind dem Fachmann an sich bekannt. Beispielsweise eignen sich wie eingangs bereits erwähnt optisch nicht oder wenig streuende, wasserunlösliche, nicht quellende, gut benetzbare, feine, anorganische oder organische Partikel, die das schnelle Eindringen auch größerer Moleküle (beispielsweise Lipide in Form von Lipoproteinen) und sogar von Zellen (z. B. Erythrozyten) in wasserfeste Filme ermöglichen. Als Beispiel für Füllstoffe seien genannt Kreide, Cellulose, Diatomeenerde, Celatom, Kieselgur, Kieselsäure, etc. Für die Zwecke der Erfindung haben sich insbesondere Celatom und Kieselgur als geeignet erwiesen.

Als Filmbildner kommen erfindungsgemäß vor allem organische Polymere in Frage, die mechanisch stabile, wasserfeste Reagenzschichten ermöglichen. Solche Filmbildner sind dem Fachmann an sich bekannt. Beispielsweise eignen sich wie eingangs bereits erwähnt Vinylpropionat-Kunststoff-Dispersionen, z. B. Propiofan^{®}, Eudragit^{®} (eine Dispersion eines Acrylharzes), Mowiol® (ein Polyvinylalkohol), und dergleichen mehr.

Erfindungsgemäß wird der Reaktivfilm auf eine mikroporöse Trägerschicht, die auch als mikroporöse Membran bezeichnet werden kann, beschichtet. Insbesondere für die Verwendung von Vollblut als Probenmaterial ist es vorteilhaft, wenn die Membran blutabtrennende Eigenschaften aufweist, d. h. in der Lage ist, aus einer Vollblutprobe die gefärbten Bestandteile (v. a. Erythrozyten, Hämoglobin) zurückzuhalten und so Plasma oder Serum aus Vollblut zu erzeugen. Solche Membranen sind dem Fachmann an sich bekannt. Beispielsweise seien genannt Polyether- oder Poylethersulfonmembranen, die vorzugsweise asymmetrisch sind. Beispielsweise seien genannt BTS SP 300 (Pall), Prime Care X oder SG (Spectral Diagnostics).

Erfindungsgemäß hat es sich als vorteilhaft herausgestellt, die mikroporöse Membran vor dem Beschichten mit der Beschichtungsmasse, die den Reaktivfilm bilden soll, zu befeuchten und im noch feuchten Zustand zu beschichten. Ganz besonders vorteilhaft ist es, die Reaktivfilmbeschichtung unmittelbar nach dem Befeuchten, d. h. wenn möglich in einem Prozessschritt, aufzubringen.

Werden Reaktivfilme auf nicht vorbefeuchtete, d.h. trockene, Membranen aufgebracht erhält man mit Reaktivfilmen beschichtete blutabtrennende Membranen, die erwartungsgemäß Erythrozyten zurückhalten. Der auf der Membran aufgebrachte Reaktivfilm füllt sich mit Plasma. Prinzipiell lässt sich auch eine von der Analytmenge abhängige Farbentwicklung beobachten. Allerdings zeigen trocken mit Reaktivfilmen beschichtete Membranen nur suboptimale Ergebnisse bei der Untersuchung von Blutproben, insbesondere wenn der Analyt ein großes oder hydrophobes Molekül (CHOL, TG, HDL etc.) ist. In diesem Fall ist das Ausmaß der Farbentwicklung im Reaktivfilm deutlich geringer als zu erwarten.

Ein Aufbringen lipidhaltiger Plasmen direkt auf diesen Reaktivfilm, d. h. ohne dass zuvor aus Vollblut über die Membran eine Blutabtrennung erfolgt ist, führt hingegen zur erwarteten Farbentwicklung. Werden die gleichen Proben zunächst durch die trocken beschichtete, blutabtrennende Membran geführt ergibt sich trotz gewährleisteter Benetzung des Reaktivfilms mit Plasma nahezu keine Farbentwicklung. Dieser experimentelle Befund lässt vermuten, dass durch ein Aufbringen des an sich relativ offenen, Füllstoffe enthaltenden Films die Durchlässigkeit der Membran für große hydrophobe Moleküle stark herabgesetzt wird.

Am Beispiel des TG-Nachweises konnte explizit gezeigt werden, dass die Durchlässigkeit kleiner, gut löslicher, intermediär entstehender Analytzwischenstufen (z. B. Glycerin, H₂O₂) durch den Membran-Reaktivfilm-Verbund, gewährleistet war (d. h. es war kein Farbunterschied zwischen Probenauftrag von oben (direkt auf den Reaktivfilm) und von unten (Probe wird auf Membran aufgegeben und durchdringt diese, bevor sie den Reaktivfilm kontaktiert) zu erkennen), selbst wenn die Membran trocken beschichtet war.

Desweiteren wurde beobachtet, dass während des Aufbringens offener, Füllstoffe und Pigmente enthaltender Filme auf saugende, nicht vorbefeuchtete Membranen die metastabilen Beschichtungsmassen begannen sich zu entmischen. Die Pigment- bzw. Füllstoff-Anteile der Beschichtungsmassen reicherten sich während des Beschichtungsvorganges am Rakelspalt an. Dies führte zu stark inhomogenen Beschichtungen.

Durch das Herabsetzen des Pigment/Füllstoffanteils in der Beschichtungspaste war es zwar möglich, stabilere Beschichtungspasten und damit homogenere Filme auf saugende, nicht vorbefeuchtete Membranen aufzubringen, die Analytdurchlässigkeit konnte dadurch jedoch nicht gefördert werden, da Filme mit geringerem Füllstoffanteil eher weniger offenporig und damit schlechter analytdurchlässig, insbesondere für große oder hydrophobe Analyten sind.

Unerwarteterweise wurde nun gefunden dass es möglich ist, feststoffhaltige, metastabile Reaktivfilme auf saugende Membranen ohne Herabsetzen der Analytpermabilität aufzubringen, wenn die Membranen im feuchten Zustand beschichtet werden.

Verfahrenstechnisch kann dies vorteilhaft durchgeführt werden, in dem die Membran z.B. in einem Prozessschritt zunächst durch ein Wasserbad geführt und anschließend die feststoffhaltige Paste auf die noch feuchte Membran mittels Rakel oder Schlitzdüse aufgebracht wird.

Ein positiver Nebeneffekt neben der gesteigerten Analytdurchlässigkeit ist das Aufbringen deutlich homogenerer Filme, da die Tendenz metastabiler feststoffhaltiger Pasten, sich während des Beschichtungsprozesses zu entmischen, bei der Verwendung feuchter Membranen deutlich herabgesetzt wird. Homogenere Filme bedeuten letztendlich bessere Präzisionen bei photometrischen Messungen und damit geringere Variationskoeffizienten bei der Konzentrationsbestimmung.

Desweiteren ist es möglich, mit diesem Verfahren offenporige Filme durch Verwendung hoher Füllstoffanteile in der Beschichtungspaste auf Membranen aufzubringen.

Da dieses Verfahren offenbar das Eindringen von Komponenten des Reaktivfilms während des Beschichtungsprozesses in Membranen minimiert, eröffnet es aufgrund der besseren räumlichen Trennung von Blutabtrennung (in der Membran) und Reaktivfilm (auf der Membran) in einem Ein-Schicht-Aufbau die Möglichkeit, dem Reaktivfilm Komponenten zuzusetzen, die für die Nachweisreaktion als ganzes förderlich sind (z. B. spezielle Netzmittel, die Lipoproteine gut lösen und Lipasen bzw. Esterasen aktivieren), ohne dadurch eine gleichzeitige netzmittelbedingte Hämolyse in der blutabtrennenden Membran zu verursachen.

Das Befeuchten der Membran erfolgt vorzugsweise durch Wannentränkung, Schlitzdüsentränkung oder Besprühen. Zum Befeuchten der Membran können Wasser oder wässrige Lösungen verwendet werden, die z. B. Puffer, Netzmittel (allgemein auch als Tenside oder Detergentien bezeichnet) zur besseren Benetzung der Membran, etc. enthalten können.

Bei dem erfindungsgemäßen Verfahren ist bei der Beschichtung von Reaktivfilmen, das Verhältnis der Massen von Filmöffner zu Filmbildner 10:1 bis 1:1. Besonders Filme mit einem Verhältnis der Massen von Filmöffner zu Filmbildner von 5:1 bis 2:1 haben sich als besonders geeignet erwiesen. Reaktivfilme mit einem solchen Massen-Verhältnis zeichnen sich durch eine relativ große Offenporigkeit aus, durch die wiederum ein Eindringen großer, hydrophober Moleküle in den Reaktivfilm erleichtert bzw. erst ermöglicht wird. Bislang war es nicht möglich, solche mit homogenen, offenporigen Filmen beschichtete Membranen herzustellen. Entsprechend beschichtete mikroporöse Membranen sind deshalb ebenfalls Gegenstand der vorliegenden Erfindung.

Das Aufbringen des Reaktivfilms erfolgt mittels an sich bekannter Verfahren wie z. B. Rakelbeschichtung, Walzenauftrag oder Schlitzdüsenbeschichtung.

Neben den erfindungsgemäß beschichteten Membranen ist ein weiterer Gegenstand der vorliegenden Erfindung ein Diagnostisches Mittel zum Nachweis von Inhaltsstoffen von Körperflüssigkeiten enthaltend eine mit einem Reaktivfilm beschichtete Membran. Körperflüssigkeiten sind dabei insbesondere Blut, Serum, Plasma, Urin, Speichel, Schweiß, etc., wobei Blut bevorzugt ist. Die nachzuweisenden Inhaltsstoffe sind die typischerweise aus Körperflüssigkeiten nachzuweisenden Analyte, insbesondere große und/oder hydrophobe Moleküle wie CHOL, TG, HDL, etc., die in Blut in Form von Lipoproteinkomplexen vorliegen, aber auch Fructosamin, Creatinkinase (CK), Glutamat-Oxalat-Transferase (GOT), Glutamat-Pyruvat-Transaminase (GPT), Amylase, Hämoglobin, Albumin.

Vorteilhaft am Gegenstand der Erfindung ist, dass es mit dem erfindungsgemäßen Verfahren erstmals möglich ist, Reaktivfilme mit hohem Füllstoffanteil (bezogen auf die Menge an Filmbildner) auf Membranen zu beschichten und so stabile, homogene offene Filme zu erzeugen. Diese sind vor allem für den Nachweis von großen, hydrophoben Analyten aus Vollblut vorteilhaft. Die Membran übernimmt dabei die Funktion der Blutabtrennung, hält also Erythrozyten und gegebenenfalls Hämoglobin zurück, so dass für den nachfolgenden Analytnachweis mittels optischer Methoden keine Störung durch die Blutfarbe erfolgt. Der intime Kontakt zwischen Membran und Nachweisfilm ist gewährleistet, so dass ein schneller und weitgehend vollständiger Übertritt des Serums/Plasmas in den Reaktivfilm erfolgt. Analytnachweise aus wenigen µl Vollblut sind möglich. Das Beschichtungsverfahren eignet sich hervorragend für automatisierte Prozesse, insbesondere für die großflächige Fertigung von auf Membranen beschichteten Reaktivfilmen, aber auch in Rollen- oder Bandprozessen.

Die Erfindung wird durch die nachfolgenden Beispiele und Figuren näher erläutert.

Figur 1 zeigt den kinetischen Messverlauf für Teststreifen, die eine "feucht-beschichtete" Membran enthalten, in Anwesenheit von Bluten mit unterschiedlichem Triglycerid-Gehalt (65, 207, 294, 494 und 728 mg/dl), wobei die relative Remission (R in %) gegen die Zeit (t in s) aufgetragen ist.

Figur 2 zeigt die relative Remission (R) bei unterschiedlichen Triglyceridkonzentrationen (c in mg/dl) in Blutproben für trocken- (2) und feucht-beschichtete (1) Membranen.

Die Ziffern und Abkürzungen in den Figuren haben die folgende Bedeutung:
- 1: Messkurve für feucht-beschichtetet Membran
- 2: Messkurve für trocken-beschichtete Membran
- R: relative Remission
- t: Zeit
- c: Konzentration

### Beispiel 1 Verfahren zum Aufbringen eines Reaktivfilmes auf eine blutabtrennende Membran und entsprechendes Testdevice zum Nachweis von Triglyceriden aus Vollblut

### 1. Herstellung der Beschichtungsmasse

a.) Gantrez-Lösung:
   Zu 58,5 g eines 85 millimolaren Phosphatpuffers (pH 7,5) werden 35,5 g Wasser gegeben. Nach Zugabe von 1,7 g MgSO₄ werden 5,2 g Gantrez S 97 (Copolymer aus Methyvinlyether und Maleinsäureanhydrid, GAF Coproration Chemical Division) in kleinen Portionen zugegeben und 3 Stunden lang bis zum vollständigen Quellen des Gantrez gerührt. Danach werden 4,5 g einer 32 %igen NaOH-Lösung zugegeben und nach weiterem 30-minütigem Rühren 0,6 g PVP (Polyvinylpyrrolidon 25.000) eingestreut und bis zum vollständigen Lösen für weitere 20 Minuten gerührt. Danach wird der pH-Wert der Ansatzmasse mit 32 %iger NaOH auf einen pH-Wert von 6,7 - 7,0 eingestellt.
b.) Symperonic-Lösung:
   In 5,3 g Wasser werden unter 20-minütigem Rühren 1,3 g Symperonic F 68 (Polyoxyethylen-co-oxypropylen, ICI) gelöst.
c.) Zu der unter a.) beschriebenen Gantrez-Lösung werden 17,0 g Propiofan 70 D (50 %ige Polymerdispersion von Vinylpropionat in Wasser, entmonomerisiert, Bezugsquelle BASF Ludwigshafen) zugegeben und nach 30-minütigem Rühren 26,5 g Celatom MW 25 (Kieselgur, CHEMAG) innerhalb von 10 Minuten eingetragen und für weitere 20 Minuten gerührt. Danach wird dem Ansatz 6,6 g der unter b.) beschriebenen Symperonic-Lösung zugegeben und nochmals für 10 Minuten gerührt.
d.) Refloblau-Lösung
   In 23,3 g 35°C warmes Wasser werden 1,7 g Refloblau (4-(4-Dimethylaminophenyl)-5-methyl-2-(3,5-dimethoxy-4-hydroxyphenyl)-imidazol-dihydrochlorid, Roche Diagnostics) unter Lichtschutz durch 15-minütiges Rühren auf dem Magnetrührer gelöst.
e.) Titandioxid/Refloblau-Teilansatz
   Unter Lichtschutz werden 22,5 g eines 85 mmolaren Phosphatpuffers (pH 7,5) vorgelegt und 4,3 g TiO₂ (RN 56, Kronos Titan) unter Verwendung eines Dissolverrührers bei 450 UPM innerhalb von 5 Minuten eingestreut und für weitere 5 Minuten nachgerührt. Abschließend wird der TiO₂-Suspension innerhalb von 5 Minuten 25 g der unter d.) angesetzten Refloblau-Lösung zugegeben und nochmals für 30 Minuten gerührt. Danach wird der TiO₂/Refloblau-Teilansatz bis zur Verwendung im Kühlschrank unter Lichtschutz aufbewahrt.
f.) ATP-Lösung
   1,7 g ATP (Adenosintriphosphat; Di-Natriumsalz) werden in 3,3 g Wasser gelöst.
g.) DONS-Lösung
   1,3 g DONS (Dioctylnatrium-Sulfosuccinat) werden in 5,3 g Aceton gelöst.
h.) MPSC-Lösung
   0,03 g MPSC (Methylphenylsemicarbazid) werden in 0,6 g 1-Methoxy-2-Propanol unter Lichtschutz gelöst.
i.) Enzym-Lösung
   In 15,9 g eines 85 millimolaren Phosphatpuffers (pH 7,5) werden nacheinander folgende Enyzme (vorliegend als Lyophilisate) gelöst, wobei die jeweilige Enzymeinwaage abhängig von der spezifischen Aktivität der verwendeten Enzymcharge ist:
   40 Kilo Units (ca. 1,8 g) Glycero-Kinase (EC 2.7.1.30 aus Bacillus stearothermophilus; Roche Diagnostics, Kat. No. 0 717 398)
   34 Kilo Units (ca. 2,4 g)Cholesterol-Esterase (EC 3.1.1.13 aus Candida cylindracea; Roche Diagnostics, Kat. No. 0 129 046)
   28,9 Kilo Units (ca. 0,12 g)Peroxidase (EC 1.11.1.7 aus Meerettich; Roche Diagnostics, Kat. No. 0 121 606)
   27,8 Kilo Units (ca. 0,44 g)L-α-Glycerolphosphat-Oxidase (EC 1.1.3.21; recombinant; Roche Diagnostics, Cat. No. 1 582 003)
j.) Zum Gantrez/Propiofan/Celatom-Ansatz aus c.) werden abschließend nachfolgende Teilansätze unter Rühren gegeben:
   6,6 g DONS-Lösung aus g.)
   5,0 g ATP-Lösung aus f.)
   51,8 g TiO₂/Refloblau-Suspension aus e.)
   7,0 g Wasser zum Nachspülen der TiO₂/Refloblau-Suspension
   11,8 g Celatom MW 25
   0,63 g MPSC-Lösung aus h.)
   20,66 g Enzym-Lösung aus i.)
   2,2 g 85 mmolarer Phosphatpuffer zum Nachspülen der Enzym-Lösung
   Nach Zugabe jeder Teillösung (mit Ausnahme des Celatoms) wird der Ansatz für 5
   Minuten gerührt. Das Celatom wird innerhalb von 15 Minuten in kleinen Portionen
   eingestreut und der Ansatz danach für weitere 20 Minuten gerührt.

   Der Gesamtansatz (ca. 250 g) wird abschließend zum Entlüften für 20 Minuten bei 300 g zentrifugiert und anschließend sich durch die Zentrifugation eventuell absetzende Feststoffanteile mittels eines Gummiwischers langsam von Hand resuspendiert.
   Danach wird die Beschichtungsmasse durch ein 140 µm Prüfsieb passiert und nochmals unter schonendem Rühren für 10 Minuten homogenisiert.
   Die Beschichtungsmasse hat die in Tabelle 1 angegebene Zusammensetzung.

| Tabelle 1: Zusammensetzung der Beschichtungsmasse | absolut | Feststoffgehalt |
|---|---|---|
| Gantrez S97 (als Filmverdicker/Quellmittel) | 5,2 g | 5,2 g |
| PVP (Polyvinylpyrrolidon) | 0,6 g | 0,6 g |
| Propiofan-Dispersion (50 %ig in Wasser) als Filmbildner | 17 g | 8,5 g |
| Celatom (als Filmöffner) | 38,3 g | 38,3 g |
| TiO₂ RN56 (als Weißpigment) | 4,3 g | 4,3 g |
| MgSO₄ | 1,7 g | 1,7 g |
| Refloblau | 1,7 g | 1,7 g |
| Methylphenylsemicarbazid | 0,03 g | 0,03 g |
| ATP (Di-Natriumsalz) | 1,7 g | 1,7 g |
| Symperonic F68 | 1,3 g | 1,3 g |
| DONS (Dioctylnatriumsulfosuccinat) | 1,3 g | 1,3 g |
| Glycero-Kinase | 40 KU | 1,8 g |
| Cholesterol-Oxidase | 34 KU | 2,4 g |
| Peroxidase | 28,9 KU | 0,12 g |
| L-α-Glycerolphosphat-Oxidase | 27,8 KU | 0,44 g |
| Aceton | 5,3 g | - |
| 1-Methoxy-2-Propanol | 0,6 g | - |
| NaOH (32 %ig) | 4,5 g | 1,4 g |
| Wasser, dest. | 152,9 g | - |
| Summe | 241,2 g | 70,8 g |

Der Feststoffgehalt der Beschichtungspaste liegt bei 29 %. Prozentualer Feststoffanteil des Filmbildners (Propiofan) bezogen auf Gesamtfeststoffanteil ist 12 %. Prozentualer Feststoffanteil des Filmöffners (Celatom) bezogen auf Gesamtfeststoffanteil ist 54 %. Das Verhältnis Filmöffner zu Filmbildner beträgt 4,5:1.

### 2. Aufbringen des Reaktivfilms auf eine blutabtrennende Membran

Mittels nachfolgend beschriebenem Verfahren wird eine blutabtrennende Membran (Typ BTS-SP-300; Art.-Nr. 955 00 12 0953 bezogen über die Firma Pall GmbH/63303 Dreieich) mit der unter Punkt 1.) hergestellten Beschichtungsmasse zur Generierung eines Reaktivfilms beschichtet.
a) Ein ca. 1 Meter langes Membranstück (BTS-SP-300) wird zunächst durch eine mit Wasser gefüllte Edelstahlwanne gezogen und danach das überschüssige, auf der Membranoberfläche stehende Wasser unter Verwendung eines Gummiabstreifers entfernt. Auf die noch feuchte Membran wird die Beschichtungsmasse aus 1.) bei einem Vorschub von 1,5 m/min und einem Rakelspalt von 150 µm aufgerakelt.
   Die derart beschichtete Membran (nachfolgend bezeichnet als "feuchtbeschichtete Membran") wird anschließend 5 Minuten bei 50° C getrocknet.
   Abschließend wird die Membran mittels Messerwelle in 4,0 mm breite Feinschnittrollen geschnitten. Die Feinschnittrollen werden bis zur weiteren Verwendung trocken gelagert.
b) Vergleichend dazu wird ein zweites Stück BTS-SP-300 mit der identischen Beschichtungsmasse beschichtet, ohne die Membran zuvor durch eine mit Wasser gefüllte Edelstahlwanne zu ziehen (nachfolgend bezeichnet als "trocken-beschichtete Membran").

### 3. Herstellung von Teststreifen-Funktionsmustern zum Nachweis von TG aus Vollblut

Auf einer 5 mm breiten und 78 mm langen Trägerfolie (Melinex) wird eine beidseitig mit doppelseitigem Klebeband beschichtete ca. 200 µm dicke Polyesterfolie (sogenannte Spacerschicht) aufgeklebt, aus der zuvor mit Hilfe eines Schneidplotters (Typ Aristomat 1310 der Firma ARISTO Graphic Systeme; 22525 Hamburg) im Abstand von 5,0 mm 1,5 mm breite, längs zum späteren Teststreifen laufende Kapillaren (Kapillarlänge 35 mm) mittels "kiss cut-Schnitt" geschnitten wurden. Auf diese Spacer-/Kapillarschicht wird ein 5 mm x 25 mm Polyester-Netz (Typ Petex 07-98/34 der Fa. Sefar / CH-9410 Heiden) mit einer Maschenweite von 250 µm geklebt, um einerseits die Kapillare nach oben zu begrenzen und um andererseits einen Proben-/Blutdurchtritt aus der Kapillare in die darüberliegende Analytnachweiszone zu gewährleisten.

Das Scrynellnetz wird dabei so auf der Spacerschicht angeordnet, dass die ersten 5 mm der Kapillare vom Netz nicht bedeckt sind und somit als Probenauftragszone genutzt werden können.

Oberhalb des Scrynellnetzes befindet sich die mittels zweier Schmelzkleberwülste seitlich befestigte, gemäß dem unter Punkt 2 beschriebenen Verfahren mit Reaktivfilm beschichtete blutabtrennende Membran (unbeschichtete, blutabtrennende Membranseite nach unten; Reaktivfilm nach oben).

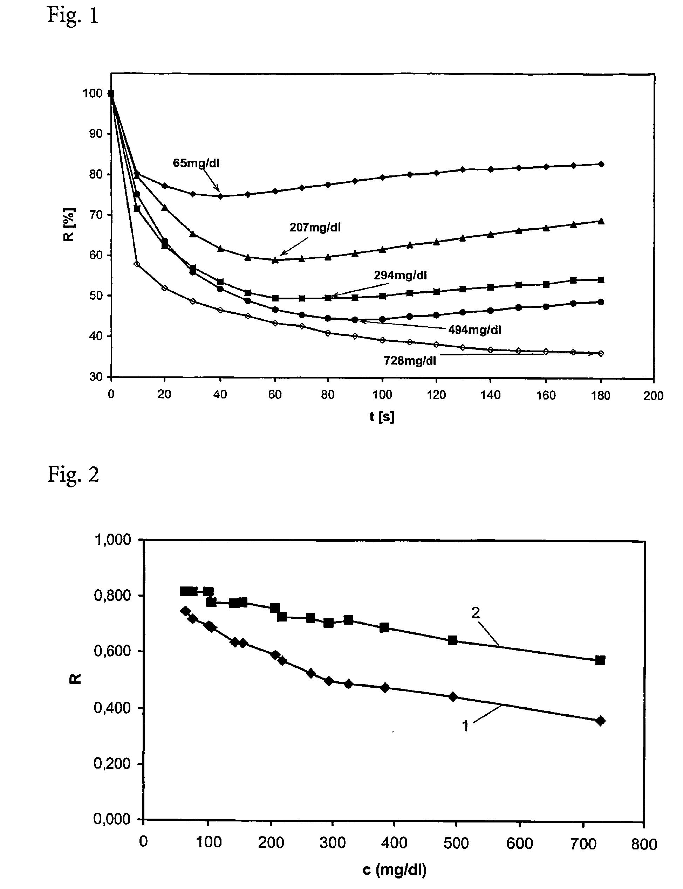

Der Aufbau des Teststreifen-Funktionsmusters ist vergleichbar mit den in Beispiel 1 und Figur 1 der EP-Anmeldung Nr. 04 023 734 (vom 5.10.2004) beschriebenen Teststreifen.

### 4. Bewertung der Funktionsmuster mit Triglyceride-enthaltenden Bluten

Auf die Teststreifen-Funktionsmuster werden 25 µl Blut in die Probenauftragszone (Kapillarbereich vor dem Scrynellnetz) appliziert. Die Muster werden über einen Zeitraum von 3 Minuten in 10 Sekunden-Intervallen mit einem optischen Messaufbau mit einer LED der Schwerpunktswellenlänge 660 nm von oben (Reaktivfilmseite der Membran) reflektionsphotometrisch vermessen.

Der Messablauf ist dabei wie nachfolgend beschrieben:
Vor Aufbringen der Probe wird der Teststreifen unter Ausschluss von Umgebungslicht einmal vermessen, um daraus die Effektivität des jeweiligen unreagierten Reaktivfilms zu erhalten. Der so erhaltene "Leerwert" des Teststreifens wird für die nachfolgende Kinetikmessung in Anwesenheit von Probenmaterial als 100 % relative Remission (R) gesetzt.

Nach Aufbringen von 25 µl Blut wird die Kinetikmessung unmittelbar gestartet. Die im Kinetikmodus erhaltenen Reflektivitäten werden durch den jeweiligen Leerwert des Teststreifens dividiert und graphisch als relative Remission (R in %) über die Messzeit aufgetragen.

Figur 1 zeigt den so erhaltenen kinetischen Messverlauf für (eine "feucht-beschichtete Membran" enthaltende) Teststreifen in Anwesenheit von Bluten mit unterschiedlichem Triglycerid-Gehalt (65, 207, 294, 494 und 728 mg/dl).

Wie den Kurvenverläufen der Kinetikmessung zu entnehmen ist, erreicht die Farbbildung des Reaktivfilms innerhalb der gewählten Messzeit konzentrationsabhängig ein Remissionsminimum (maximale Farbtiefe), wobei dieses Remissionsminimum nachfolgend als Maß der Analytkonzentration in der Probe gewählt wird.

In der folgenden Tabelle 2 sind die relativen Remissionsminima (in %) für eine mit identischem Reaktivfilm "feucht-beschichtete" und "trocken-beschichtete" BTS-SP-300 Membran aufsteigend gegen Blute mit unterschiedlichem Triglycerid-Gehalt aufgelistet.

**Tabelle 2**

| | **% relative Remission (im Minimum)** | |
|---|---|---|
| **Triglycerid-Gehalt in der Blutprobe** | **"feucht-beschichtete" Membran** | **"trocken-beschichtete" Membran** |
| 65 mg/dl | 74,7 % | 81,6 % |
| 76 mg/dl | 71,7 % | 81,5 % |
| 100 mg/dl | 69,4 % | 81,3 % |
| 105 mg/dl | 68,7 % | 77,7 % |
| 142 mg/dl | 63,5 % | 77,3 % |
| 154 mg/dl | 63,1 % | 77,8 % |
| 207 mg/dl | 59,0 % | 75,7 % |
| 217 mg/dl | 56,9 % | 72,6 % |
| 265 mg/dl | 52,6 % | 72,3 % |
| 294 mg/dl | 49,7 % | 70,3 % |
| 326 mg/dl | 48,8 % | 71,5 % |
| 384 mg/dl | 47,3 % | 68,6 % |
| 494 mg/dl | 44,2 % | 64,3 % |
| 728 mg/dl | 36,1 % | 57,5 % |
| Gesamtremissionshub | 38,6 % | 24,1 % |

Wie man Tabelle 2 entnehmen kann, ergeben die Funktionsmuster mit der "feucht-beschichteten" Membran über den gesamten Messbereich hinweg deutlich mehr Farbe (niedrigere Remissionswerte) als die mit identischem Reaktivfilm "trocken-beschichtete" Membran.

Desweiteren ist den Messwerten zu entnehmen, dass der über den gesamten Messbereich erzielte Remissionshub (d. h. die Differenz der relativen Remissionen für die Trigylceridkonzentrationen 65 mg/dl und 728 mg/dl) für die "feucht-beschichtete" Membran mit 38,6 % REM deutlich größer ist als der Remissionshub der "trockenbeschichteten" Membran mit 24,1 % REM (siehe dazu auch den graphischen Verlauf, dargestellt in Figur 2, wobei 1 die Messkurve für die feucht-beschichtete, 2 die Messkurve für die trocken-beschichtete Membran wiedergeben).

Aufgrund des deutlich größeren Remissionshubs der "feucht-beschichteten" Membran machen sich Remissionsschwankungen von Messung zu Messung in einer deutlich geringeren Konzentrationsschwankung und damit in einer höheren Präzision der Funktionsmuster bemerkbar.

## Patentansprüche

1. Verfahren zum Aufbringen feststoffhaltiger Reaktivfilme auf mikroporöse, saugende , blutabtrennende Membranen, wobei der feststoffhaltige Reaktivfilm ein wasserfester, wasserunlöslicher Film ist, der in einer hydrophoben Matrix aus Filmbildnern alle oder zumindest Teile der Nachweisreagenzien für einen nachzuweisenden Analyten enthält und einen hohen Anteil von Filmöffnern, bezogen auf die Menge an Filmbildner, aufweist, **dadurch gekennzeichnet, dass** die Filmöffner optisch nicht oder wenig streuende, wasserunlösliche, nicht quellende, gut benetzbare, feine, anorganische oder organische Partikel sind, welche das schnelle Eindringen auch größerer Moleküle oder Zellen in den feststoffhaltigen Reaktivfilm ermöglichen und welche in einem Verhältnis der Massen von Filmöffner zu Filmbildner von 10:1 1 bis 1:1 im Reaktivfilm vorliegen und die Membran zunächst mit Wasser oder einer wässrigen Lösung befeuchtet und der feststoffhaltige Reaktivfilm auf die noch feuchte Membran aufgebracht wird.

2. Verfahren gemäß Anspruch 1, **dadurch gekennzeichnet, dass** der Reaktivfilm Filmbildner, anorganische oder organische partikelförmige Filmöffner und gegebenenfalls optisch sperrende Pigmente enthält.

3. Verfahren gemäß einem der vorigen Ansprüche, **dadurch gekennzeichnet, dass** der Reaktivfilm unmittelbar nach dem Befeuchten auf die Membran aufgebracht wird.

4. Verfahren gemäß einem der vorigen Ansprüche, **dadurch gekennzeichnet, dass** das Befeuchten der Membran durch Wannentränkung erfolgt.

5. Verfahren gemäß einem der vorigen Ansprüche, **dadurch gekennzeichnet, dass** das Wasser oder die wässrige Lösung ein Netzmittel enthält.

6. Verfahren gemäß einem der vorigen Ansprüche, **dadurch gekennzeichnet, dass** das Aufbringen des Reaktivfilms mittels Rakelbeschichtung oder mittels Schlitzdüsenbeschichtung erfolgt.

7. Mikroporöse, saugende , blutabtrennende Membran, die mit einem feststoffhaltigen Reaktivfilm beschichtet ist, wobei der feststoffhaltige Reaktivfilm ein wasserfester, wasserunlöslicher Film ist, der in einer hydrophoben Matrix aus Filmbildnern alle oder zumindest Teile der Nachweisreagenzien für einen nachzuweisenden Analyten enthält und optisch nicht oder wenig streuende, wasserunlösliche, nicht quellende, gut benetzbare, feine, anorganische oder organische Partikel als Filmöffner enthält, welche das schnelle Eindringen auch größerer Moleküle oder Zellen in den feststoffhaltigen Reaktivfilm ermöglichen, **dadurch gekennzeichnet, dass** das Massen-Verhältnis von Filmöffner zu Filmbildner 10:1 bis 1:1 ist.

8. Membran gemäß Anspruch 7 , **dadurch gekennzeichnet, dass** das Massen-Verhältnis von Filmöffner zu Filmbildner 5:1 bis 2:1 ist.

9. Diagnostisches Mittel zum Nachweis von Inhaltsstoffen von Körperflüssigkeiten enthaltend eine mit einem Reaktivfilm beschichtete Membran gemäß einem der Ansprüche 7 bis 8.

## Claims

1. Method for the application of reactive films containing solids onto microporous, absorbent, blood-separating membranes, where the reactive film containing solids is a water-resistant, water-insoluble film which contains all or at least some of the detection reagents for an analyte to be detected in a hydrophobic matrix made of film formers and a high proportion of film openers based on the amount of film formers, **characterized in that** the film openers are optically non-scattering or slightly scattering, water-insoluble, non-swelling, readily wettable, fine, inorganic or organic particles which enable even relatively large molecules or cells to rapidly penetrate into the reactive film containing solids and which are present in the reactive film in a ratio of the masses of film opener to film former of 10:1 1 to 1:1 and the membrane is firstly moistened with water or an aqueous solution and the reactive film containing solids is applied to the still moist membrane.

2. Method according to claim 1, **characterized in that** the reactive film contains film formers, inorganic or organic particulate film openers and optionally optically blocking pigments.

3. Method according to one of the previous claims, **characterized in that** the reactive film is applied to the membrane immediately after the moistening.

4. Method according to one of the previous claims, **characterized in that** the moistening of the membrane is carried out by bath impregnation.

5. Method according to one of the previous claims, **characterized in that** the water or the aqueous solution contains a wetting agent.

6. Method according to one of the previous claims, **characterized in that** application of the reactive film is carried out by means of knife coating or by means of slot nozzle coating.

7. Microporous, absorbent, blood-separating membrane which is coated with a reactive film containing solids, where the reactive film containing solids is a water-resistant, water-insoluble film which contains all or at least some of the detection reagents for an analyte to be detected in a hydrophobic matrix made of film formers and contains optically non-scattering or slightly scattering, water-insoluble, non-swelling, readily wettable, fine, inorganic or organic particles as film openers, which enable even relatively large molecules or cells to penetrate rapidly into the reactive film containing solids, **characterized in that** the mass ratio of film opener to film former is 10:1 1 to 1:1.

8. Membrane according to claim 7, **characterized in that** the mass ratio of film opener to film former is 5:1 to 2:1.

9. Diagnostic agent for detecting components of body fluids containing a membrane coated with a reactive film according to one of the claims 7 to 8.

## Revendications

1. Procédé pour appliquer des films réactifs contenant une ou plusieurs substances solides sur des membranes microporeuses, absorbantes, pour la séparation du sang, le film réactif contenant une ou plusieurs substances solides représentant un film insoluble dans l'eau, résistant à l'eau, qui contient, dans une matrice hydrophobe constituée par des agents filmogènes, la totalité ou au moins des parties des réactifs de décèlement pour un analyte à déceler et qui présente une fraction élevée d'ouvreurs de films, rapportée à la quantité d'agents filmogènes, **caractérisé en ce que** les ouvreurs de films représentent de fines particules inorganiques ou organiques, manifestant une bonne aptitude au mouillage, qui ne gonflent pas, qui sont insolubles dans l'eau et qui ne dispersent pas ou qui dispersent peu la lumière, lesdites particules permettant une pénétration rapide également de molécules ou de cellules relativement grosses dans le film réactif contenant une ou plusieurs substances solides et étant présentes dans le film réactif dans un rapport massique des ouvreurs de films aux agents filmogènes de 10:1 à 1:1, et **en ce que** la membrane est humidifiée dans un premier temps avec de l'eau ou avec une solution aqueuse et le film réactif contenant une ou plusieurs substances solides est appliqué sur la membrane encore humide.

2. Procédé selon la revendication 1, **caractérisé en ce que** le film réactif contient des agents filmogènes, des ouvreurs de films particulaires inorganiques ou organiques et le cas échéant des pigments ayant un effet de blocage optique.

3. Procédé selon l'une quelconque des revendications précédentes, **caractérisé en ce que** le film réactif est appliqué sur la membrane directement après l'humidification.

4. Procédé selon l'une quelconque des revendications précédentes, **caractérisé en ce que** l'humidification de la membrane a lieu par imprégnation en cuve.

5. Procédé selon l'une quelconque des revendications précédentes, **caractérisé en ce que** l'eau ou la solution aqueuse contient un agent mouillant.

6. Procédé selon l'une quelconque des revendications précédentes, **caractérisé en ce que** l'application du film réactif a lieu au moyen d'une enduction à la racle ou au moyen d'une enduction à filière plate.

7. Membrane microporeuse, absorbante, pour la séparation du sang, qui est enduite d'un film réactif contenant une ou plusieurs substances solides, le film réactif contenant une ou plusieurs substances solides représentant un film insoluble dans l'eau, résistant à l'eau, qui contient, dans une matrice hydrophobe constituée par des agents filmogènes, la totalité ou au moins des parties des réactifs de décèlement pour un analyte à déceler et qui contient, à titre d'ouvreurs de films, des fines particules inorganiques ou organiques, manifestant une bonne aptitude au mouillage, qui ne gonflent pas, qui sont insolubles dans l'eau et qui ne dispersent pas ou qui dispersent peu la lumière, lesdites particules permettant une pénétration rapide également de molécules ou de cellules relativement grosses dans le film réactif contenant une ou plusieurs substances solides, **caractérisée en ce que** le rapport massique des ouvreurs de films aux agents filmogènes s'élève de 10:1 à 1:1.

8. Membrane selon la revendication 7, caractérisée le rapport massique des ouvreurs de films aux agents filmogènes s'élève de 5:1 à 2:1.

9. Agent diagnostique pour le décèlement de constituants de liquides corporels, contenant une membrane enduite d'un film réactif, selon l'une quelconque des revendications 7 à 8.
